# FASCICULE DE BREVET EUROPEEN

(11) **EP 1 959 778 B1**
(45) Date de publication et mention de la délivrance du brevet: **02.11.2011**
(21) Numéro de dépôt: 06841925.8
(22) Date de dépôt: 13.12.2006
(51) Int. Cl.: A43B 13/12, A43B 13/14, A43B 17/18, A43B 7/14, A61F 5/01

(54) **CHAUSSURE THERAPEUTIQUE**
THERAPEUTISCHER SCHUH
THERAPEUTIC SHOE

(30) Priorité: 14.12.2005 FR 0512671
(43) Date de publication de la demande: 27.08.2008
(73) Titulaire: DJO France, 64990 Mouguerre (FR)
(72) Inventeur: DETERME, Patrice, F-31400 Toulouse (FR); LAFFENETRE, Olivier, F-33000 Bordeaux (FR); CERMOLACCE, Christophe, F-13008 Marseille (FR); COILLARD, Jean-Yves, F-69450 Saint Cyr au Mont d'Or (FR)
(74) Mandataire: Cabinet BARRE LAFORGUE & associés
(86) Numéro de dépôt international: PCT/FR2006/002721
(87) Numéro de publication internationale: WO 2007/068827

(56) Documents cités:
- EP-A- 1 488 715
- DE-U1- 29 709 409
- US-A- 5 491 909
- US-B1- 6 277 087
- US-B1- 6 485 447

## Description

L'invention concerne une chaussure thérapeutique comportant un semelage présentant une surface d'appui au sol à profil bombé dans un plan sagittal de la chaussure et une surface supérieure comprenant un emplacement pour orteils et un emplacement pour talon.

L'invention concerne en particulier les chaussures postopératoires et post-traumatiques.

On connaît déjà des chaussures thérapeutiques munies d'une semelle rigide dont une portion présente un profil bombé dans un plan sagittal de la chaussure visant à permettre, lors de la marche, un basculement progressif, du talon vers l'avant du pied du porteur. Par rapport à une semelle rigide plate, une telle semelle facilite la marche et permet un transfert plus progressif du poids du corps du talon vers l'avant du pied pendant la foulée. Ces chaussures sont adaptées pour prévenir qu'une pression soit exercée sur les orteils du porteur à la fin de la foulée.

Ces chaussures sont notamment employées pour empêcher toute flexion de l'avant du pied défavorable à la guérison suite à une intervention chirurgicale. Ces chaussures permettent en particulier de prévenir la mobilisation des phalanges par rapport aux métatarses après une opération correctrice d'un HALLUX VALGUS. Ces chaussures permettent de prévenir toute flexion des orteils par rapport aux métatarsiens.

Or, il existe un besoin d'augmenter le confort du patient en permettant une flexion dosée du pied pendant la marche lorsqu'il est muni de la chaussure thérapeutique, en particulier au niveau des jointures des métatarses et des phalanges.

Par ailleurs, l'immobilisation rigide du pied est suivie le plus souvent d'une période de rééducation pendant laquelle le patient est amené à retrouver progressivement l'usage normal de son pied, ou du moins à retrouver une partie des possibilités de mouvements du pied en flexion. La chaussure thérapeutique est alors retirée pour permettre l'exécution, sous l'assistance d'un physiothérapeute, de mouvements en flexion du pied permettant cette rééducation.

EP 1 488 715, divulguant une chaussure selon le préambule de la revendication 1, propose une chaussure postopératoire évolutive pouvant être adaptée aux différentes phases consécutives à l'opération jusqu'à ce que le patient puisse reprendre un chaussage classique. Cette chaussure postopératoire comprend un corps de base avec une semelle semi-rigide, et un ensemble d'accessoires démontables et interchangeables sous la semelle, notamment une plaque métallique de rigidification et un tampon de forme arrondie. Durant une première phase de rééducation qui suit immédiatement l'intervention chirurgicale du pied, la chaussure post-opératoire est rigidifiée au moyen de la plaque métallique appliquée et fixée contre la face inférieure de la semelle du corps de base. Durant cette phase, le tampon est fixé sous la plaque métallique. Lors d'une deuxième phase de rééducation, la plaque métallique et le tampon notamment sont démontés afin de rendre sa souplesse initiale à la semelle du corps de base. Cette chaussure ne peut pas être aisément adaptée par le patient lui-même en fonction des différentes phases de rééducation. En outre, les inventeurs ont déterminé qu'elle ne répond pas de façon appropriée aux réels besoins d'immobilisation et de mobilisation rencontrés dans chacune des phases.

À cet égard, les inventeurs ont déterminé qu'il existe un besoin de permettre aux patients d'effectuer eux-mêmes cette rééducation, notamment pendant la réalisation d'activités courantes faites par le patient. Pour ce faire, il convient de proposer des chaussures thérapeutiques favorisant la réalisation avec amplitude réduite des flexions du pied se produisant naturellement pendant la marche En particulier, il faut pouvoir faire réaliser par le patient, naturellement pendant la foulée, une mobilisation des phalanges par rapport aux métatarses avec une amplitude de mouvement de ces os réduite par rapport à une mobilisation se produisant pendant la foulée lorsque le pied est chaussé de chaussures à fond plat.

Par ailleurs, les inventeurs ont déterminé qu'il convient de permettre au patient de choisir le moment pour réaliser cette rééducation en lui permettant à tout moment d'autoriser ou d'interdire lui-même les flexions du pied correspondantes. En particulier, il s'agit de permettre au patient d'immobiliser son pied en réponse à une sensation d'inconfort, de douleur, de fatigue, ...

La présente invention vise à proposer une chaussure thérapeutique répondant aux besoins décrits précédemment.

Plus particulièrement, elle vise à proposer une telle chaussure thérapeutique de prix de revient réduit permettant de réaliser de façon autonome une telle rééducation.

En outre, elle vise à proposer une telle chaussure thérapeutique dont l'utilisation n'est pas susceptible de faire l'objet d'une contre indication médicale dans le cadre de la cicatrisation du pied suite à un acte chirurgical ou du traitement de la plupart des blessures et affections du pied pour lesquelles une rééducation telle que décrite précédemment est préconisée.

Elle vise encore à proposer une telle chaussure thérapeutique qui soit simple d'utilisation, notamment quant aux manipulations à effectuer pour autoriser, interdire, ou ajuster les possibilités de la flexion du pied.

Pour ce faire l'invention concerne une chaussure thérapeutique selon la revendication 1.

La chaussure thérapeutique selon l'invention utilisée sans pièce rigide comporte l'avantage, par rapport aux chaussures thérapeutiques traditionnelles à semelages semi-rigides et à surfaces d'appui au sol plates utilisées traditionnellement pendant la phase de rééducation, de permettre à l'utilisateur de réaliser une foulée naturelle avec une flexion réduite du pied, donc moins douloureuse et/ou inconfortable, de sorte que la rééducation du pied peut s'effectuer plus progressivement et plus naturellement pour le patient.

Le mouvement de la rééducation correspondant à un mouvement naturel pour le patient, ce dernier est susceptible de pouvoir le réaliser de manière autonome.

Toujours dans le cas où la chaussure thérapeutique est utilisée sans pièce rigide, la chaussure thérapeutique selon l'invention permet de réaliser, pendant une phase finale du déroulement du pied pendant la foulée, un transfert de poids sur l'avant pied produisant une flexion progressive du pied, au niveau des jointures des métatarses et des phalanges du pied, d'amplitude souhaitée.

Par ailleurs, dans le cas où la chaussure est utilisée munie de la (des) pièce(s) rigide(s), la chaussure thérapeutique permet un déroulement progressif du pied depuis le talon jusqu'aux orteils, facilitant ainsi la réalisation de la foulée.

Avantageusement et selon l'invention, la surface d'appui au sol est exclusivement constituée par une surface d'usure d'une semelle d'usure formée en une pièce unique et s'étend en regard d'une extrémité postérieure de l'emplacement pour talon, jusqu'en regard d'une extrémité antérieure de l'emplacement pour orteils.

Avantageusement et selon l'invention, le profil bombé de la surface d'appui s'étend jusqu'en regard d'une extrémité antérieure de l'emplacement pour orteils.

Avantageusement et selon l'invention, au moins une pièce rigide est conformée pour s'étendre en regard de l'emplacement pour talon et de l'emplacement pour orteils.

De préférence, au moins une pièce rigide est conformée pour s'étendre jusqu'en regard d'une extrémité antérieure de l'emplacement pour orteils. Ainsi la chaussure thérapeutique est adaptée pour s'opposer à une flexion du pied au niveau des jointures des phalanges et des métatarses.

Avantageusement et selon l'invention, le semelage est fixé de manière inamovible à une tige de la chaussure thérapeutique.

Avantageusement et selon l'invention, ladite surface d'appui est formée par une semelle d'usure du semelage formant un revêtement extérieur inférieur du semelage.

Avantageusement et selon l'invention, au moins une pièce rigide de renfort est logée de façon amovible dans un logement du semelage de forme conjuguée de celle de cette pièce rigide de renfort. Avantageusement et selon l'invention, ce logement est formé dans une semelle d'usure du semelage et/ou dans une semelle intérieure amovible du semelage reposant au moins en partie sur une face intérieure supérieure de la semelle d'usure, qui est opposée à ladite surface d'appui au sol. Avantageusement et selon l'invention, ledit logement est formé d'un évidement de ladite face intérieure de la semelle d'usure. Avantageusement et selon l'invention, ledit logement est formé dans une première de la chaussure (dans tout le texte, le terme « première » désigne une semelle supérieure de la chaussure destinée à recevoir le pied de l'utilisateur et présentant donc ladite surface supérieure du semelage formant l'emplacement pour orteils et l'emplacement pour talon).

Avantageusement et selon l'invention, le semelage comporte des moyens de fixation amovible de la semelle intérieure par rapport à la face intérieure de la semelle d'usure, adaptés pour s'opposer à tout glissement longitudinal relatif de la semelle intérieure par rapport à la face intérieure de la semelle d'usure.

Avantageusement et selon l'invention, la semelle d'usure présente au moins un plot s'étendant vers le haut, chaque plot étant adapté pour pénétrer dans une cavité ménagée dans ladite première, le(les) plot(s) et la(les) cavité(s) présentant des formes conjuguées pour former des moyens de fixation amovible.

Avantageusement et selon l'invention, chaque pièce rigide de renfort est formée d'une résine chargée de fibre de verre.

Dans un mode de réalisation préférentiel, avantageusement et selon l'invention, la chaussure comprend une unique pièce rigide de renfort amovible sous forme d'une plaque s'étendant longitudinalement et horizontalement en regard de la majeure partie de ladite surface d'appui. Avantageusement et selon l'invention, cette plaque s'étend aussi en regard de la majeure partie de la surface supérieure du semelage. Avantageusement et selon l'invention, cette plaque rigide de renfort s'étend longitudinalement depuis l'emplacement pour talon jusqu'à l'emplacement pour orteils -notamment jusqu'à l'extrémité antérieure de l'emplacement pour orteils- . En variante, il peut être prévu une plaque rigide de renfort qui s'étend longitudinalement sous l'emplacement pour orteils mais sans atteindre l'extrémité antérieure de cet emplacement pour orteils. Une même chaussure selon l'invention peut d'ailleurs être proposée avec deux plaques rigides de renfort de longueurs et/ou formes différentes, pouvant être substituées l'une à l'autre, le patient choisissant la plaque de renfort appropriée selon l'état de sa rééducation. De préférence, ladite plaque est cintrée selon un profil arqué avec une courbure dans le même sens que le profil bombé de ladite surface d'appui. De préférence et selon l'invention, la courbure de ladite plaque est au moins sensiblement conjuguée de celle du profil bombé de ladite surface d'appui, c'est-à-dire présente une forme de courbure similaire.

L'invention concerne également une chaussure thérapeutique caractérisée en combinaison par tout ou partie des caractéristiques mentionnées ci-dessus ou ci-après.

D'autres caractéristiques, buts et avantages de l'invention apparaîtront à la lecture de la description suivante qui se réfère aux figures annexées dans lesquelles :
- la figure 1 est une vue schématique de côté d'une chaussure selon un premier mode de réalisation de l'invention,
- la figure 2 est une vue schématique éclatée et en coupe d'un semelage de la chaussure de la figure 1, selon un plan sagittal de la chaussure,
- la figure 3 est une vue schématique de dessus d'une semelle d'usure du semelage de la figure 2,
- la figure 4 est une vue schématique de dessus d'une pièce rigide de renfort amovible du semelage de la figure 2,
- la figure 5 est une vue schématique en perspective d'une semelle d'usure utilisée pour réaliser une chaussure thérapeutique selon un deuxième mode de réalisation de l'invention.

Dans ce qui suit, les directions et les dimensions doivent s'interpréter, sauf indications contraires, selon une représentation de la chaussure vue de face, chaussée et reposant sur un sol horizontal, une surface d'appui de la chaussure thérapeutique en contact avec le sol.

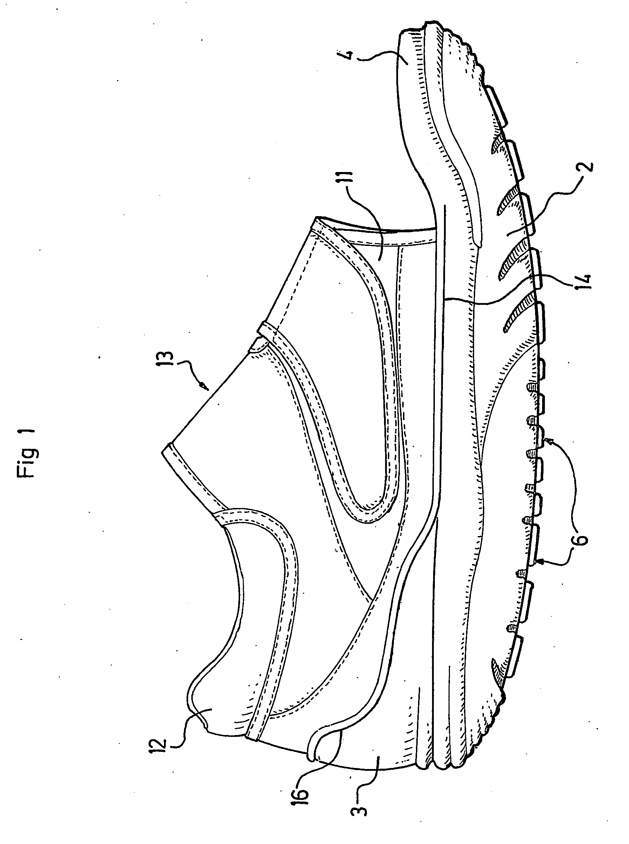

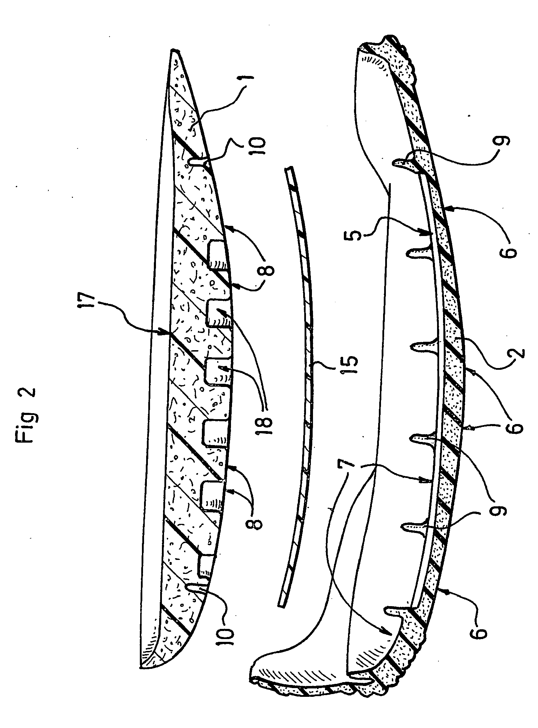

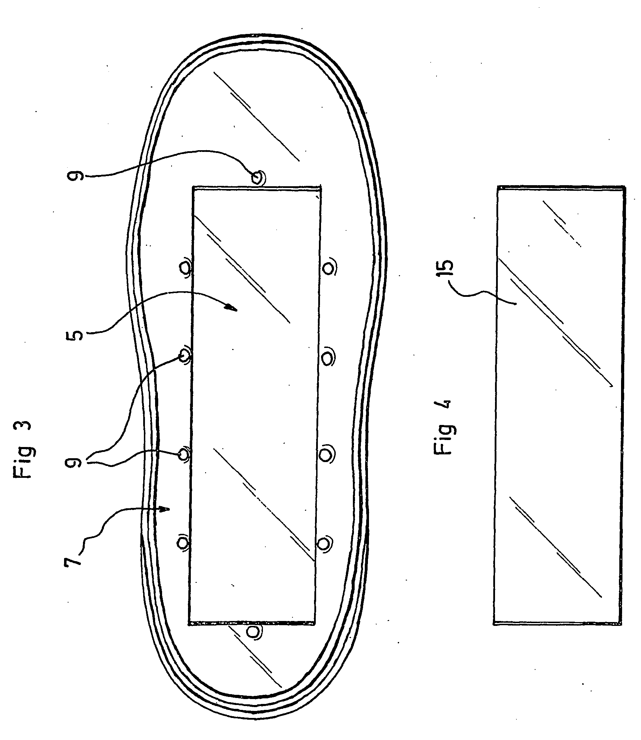

Dans le mode préféré de réalisation représenté figures 1 à 4, la chaussure thérapeutique selon l'invention comporte une tige et un semelage assemblé solidaire à la tige.

La tige est formée d'un assemblage de pièces textiles formant notamment deux quartiers adaptés pour recouvrir, lorsque la chaussure est portée, le cou-de-pied et le talon du porteur.

Les quartiers de la tige sont adaptés pour pouvoir s'écarter de manière à permettre une insertion verticale dans la chaussure et une extraction verticale hors de la chaussure du pied du porteur. Les quartiers s'étendent depuis le semelage pour se rejoindre par-dessus le cou-de-pied du porteur lors de la fermeture de la chaussure. Chaque quartier 11 comporte une bride 13 prolongeant le quartier 11 depuis la jonction des deux quartiers 11 au-dessus du cou-de-pied du porteur pour se rabattre, lors de la fermeture de la chaussure, par-dessus l'autre quartier 11. La chaussure comporte aussi des moyens d'attache par boucles et crochets adaptés pour permettre une fermeture de la chaussure par application d'une face de la bride 13 de chaque quartier 11 sur une face extérieure de l'autre quartier 11. Une des brides prolonge une portion supérieure du quartier 11 correspondant au niveau du cou-de-pied, tandis que l'autre bride 13 prolonge une portion inférieure du quartier 11 correspondant au niveau du cou-de-pied.

De préférence et comme représenté, les quartiers de la tige s'étendent en-dessous de la malléole du porteur lorsque la chaussure est portée. Un rebord de la tige à proximité de la malléole présente un matelassage 12 par souci de confort. De même, l'intérieur de la tige peut être recouvert d'un matelassage par souci de confort.

Dans le premier mode de réalisation représenté, la tige de la chaussure ne comporte pas de claque avant (empeigne) de sorte que la chaussure présente, disposée en avant de la chaussure, une ouverture. Une telle ouverture permet, lorsque la chaussure est portée, un accès aux orteils du porteur sans avoir à retirer la chaussure.

En pratique, les pièces de textile formant les quartiers 11 de la tige peuvent être formées de tout textile apte à pouvoir maintenir le pied en position par rapport au semelage lors de la marche. D'autres matériaux que les textiles peuvent à l'évidence être employés, par exemple du cuir, ou tout autre matériau approprié, notamment un matériau léger par souci de procurer un confort de marche satisfaisant.

La tige telle que décrite permet de stabiliser la cheville et l'ensemble de l'arrière pied.

De préférence et comme représenté, le semelage comporte une semelle 2 d'usure et une première 1 destinée à recevoir le pied du patient.

La première 1 comporte une face 8 inférieure à profil bombé dans un plan sagittal du semelage.

En outre, ladite première 1 comporte une face 17 supérieure sur laquelle le pied du porteur repose lorsque la chaussure est portée. Cette face 17 supérieure peut être spécifiquement conformée selon le traitement à réaliser et les contraintes ergonomiques liées à ce traitement. A titre d'exemple, tel qu'illustré dans les figures, la face 17 supérieure de ladite première 1 peut être globalement plane.

En outre, la face 8 inférieure de ladite première 1 comporte des évidements 18 de façon à diminuer le poids de ladite première 1.

La première 1 est par exemple formée en chlorure de polyvinyle expansé. En pratique, ladite première 1 peut être découpée dans un bloc de chlorure de polyvinyle expansé. Ladite première 1 peut-être faite de tout autre matériau semi-rigide adapté à une utilisation médicale, notamment un matériau léger par souci de procurer un confort de marche satisfaisant.

Il est à noter que l'on désigne par l'expression "semi-rigide" la qualité d'un matériau de comporter des propriétés mécaniques sous contraintes (rigidité, élasticité en particulier) telles que le matériau est apte à permettre, sous les contraintes transmisses par le pied lors de la foulée dans des conditions normales de marche, une flexion du semelage se traduisant par une flexion du pied sensible pour le porteur.

La première 1 peut être faite de tout matériau léger adapté à un usage médical et présentant un comportement semi-rigide en flexion adapté pour pouvoir former une première 1 autorisant des flexions d'amplitudes réduites du pied pendant la marche lorsque la chaussure est utilisée sans pièce 15 rigide de renfort amovible.

En outre, ladite première 1 peut être obtenue par tout autre procédé de formage permettant de lui conférer la forme souhaitée. En particulier, ladite première peut être moulée, extrudée, ...

Dans le premier mode de réalisation, la semelle 2 d'usure forme un revêtement inférieur de toute la face 8 inférieure de ladite première 1, épousant cette face 8 inférieure. Ainsi, la surface 6 d'appui au sol de la semelle 2 d'usure et la face 8 inférieure présentent des profils similaires.

La semelle 2 d'usure présente une surface 6 d'appui au sol à profil bombé convexe dans le plan sagittal du semelage.

De préférence, la surface 6 d'appui présente un profil régulier convexe et symétrique de part et d'autre du plan sagittal selon des plans de coupe parallèles au plan sagittal de façon à procurer une stabilité latérale du pied du patient et à permettre une utilisation pour le pied gauche comme pour le pied droit.

Dans le premier mode préféré de réalisation, la semelle 2 d'usure comporte une face 7 intérieure supérieure concave contre laquelle la face 8 inférieure convexe de ladite première 1 vient en contact. Cette face 7 intérieure comporte un évidement 5 conformé pour loger une barrette 15 rigide présentant, selon une section sagittale de la chaussure, un profil arqué dont la courbure épouse le profil bombé de la face 8 inférieure de ladite première 1. De préférence, l'évidement 5 et la barrette 15 rigide sont de formes conjuguées.

La barrette 15 peut être formée de tout matériau rigide tel qu'un métal, un polymère, un matériau composite... A titre d'exemple, ce matériau peut être du polyester chargé de fibre de verre. La barrette 15 rigide peut être faite de tout autre matériau rigide adapté à une utilisation médicale, notamment dans un matériau léger par souci de procurer un confort de marche satisfaisant.

En outre, la face 7 intérieure de la semelle d'usure 2 présente avantageusement des ergots (non représentés) disposés en bordure de l'évidement 5 s'étendant en saillie horizontalement vers l'intérieur de cette bordure et permettant de maintenir la barrette 15 rigide dans l'évidement 5 suite à son insertion dans cet évidement 5. En alternative, les ergots peuvent être remplacés par un débordement de la bordure de l'évidement 5.

Dans le premier mode préféré de réalisation, l'évidement 5 est disposé sur la face 7 intérieure de la semelle 2 d'usure de manière à s'étendre longitudinalement selon un plan sagittal de la chaussure. L'évidement 5 et la barrette 15 rigide sont de préférence adaptés pour s'étendre longitudinalement lorsque la chaussure est portée avec la pièce 15 rigide logée dans le semelage, sous le pied du porteur, cette pièce 15 rigide s'étendant en regard du talon et se prolongeant vers l'avant pied jusqu'en regard des orteils du porteur et de préférence jusqu'en regard de l'extrémité antérieure des orteils. Dès lors, la chaussure thérapeutique ainsi portée prévient la flexion intempestive du pied au niveau de jointures des phalanges et des métatarses.

Dans le premier mode de réalisation préféré de l'invention, la semelle 2 d'usure présente des plots 9 s'étendant vers le haut depuis la face 7 intérieure de la semelle 2 d'usure pour pénétrer des cavités 10 conjuguées ménagées dans la face 8 inférieure de ladite première 1. Les plots 9 sont disposés autour de l'évidement 5. Ces plots 9 facilitent l'assemblage et permettent de prévenir tout glissement longitudinal de ladite première 1 sur la face 7 intérieure de la semelle 2 d'usure. Les plots 9 forment aussi des moyens de fixation amovible de la semelle intérieure 1 sur la face intérieure 7 de la semelle 2 d'usure. Il est à noter que ces moyens de fixation amovible peuvent être réalisés par tous autres moyens de fixation amovible appropriés, tels que : d'autres moyens de fixation par encastrements relatifs, des moyens de fixation à boucles et crochets, des brides d'attaches de ladite première sur la semelle d'usure etc.

De préférence, les moyens de fixation amovibles sont structurellement adaptés pour ne pas significativement influer sur la flexibilité du semelage, cette dernière résultant essentiellement de la forme et du choix des matériaux de la semelle d'usure et de ladite première.

Dans le premier mode préféré de réalisation, le semelage est assemblé à la tige au moyen d'une piqûre 14 s'étendant le long d'une bordure (non représentée) inférieure des deux quartiers 11 de la tige, traversant cette bordure et l'épaisseur de la semelle 2 d'usure.

En outre, une piqure 16 s'étend de préférence le long d'une bordure d'un contrefort de la chaussure formé par un prolongement de la semelle 2 d'usure recouvrant une portion de la tige au niveau du talon porteur. La semelle 2 d'usure présente aussi un prolongement s'étendant en regard des orteils du porteur. Ce prolongement forme un pare-choc 4.

Dans le mode de réalisation de l'invention représenté, il est possible d'accéder à la barrette 15 rigide cambrée de la chaussure après avoir écarté les quartiers 11 de la tige et après avoir retiré ladite première 1. Une pression exercée sur la surface 6 d'appui de la semelle 2 d'usure permet alors d'extraire la barrette 15 cambrée de l'évidement 5.

Lorsqu'elle est portée sans la barrette 15 rigide et après avoir replacé ladite première 1 sur la face 7 intérieure de la semelle 2 d'usure, la chaussure thérapeutique autorise une flexion du pied tout en facilitant un déroulement du pied. L'amplitude en flexion procurée par la chaussure lors de la foulée dépend de la rigidité structurelle globale de la chaussure sans la pièce 15 de renfort. Elle dépend tout particulièrement de la flexibilité de ladite première 1 et de la semelle 2 d'usure.

La chaussure thérapeutique selon le mode préféré de réalisation de l'invention permet d'obtenir un support rigide en flexion du pied du porteur lorsque la barrette 15 rigide cambrée est logée dans l'évidement 5, ou un support plus souple en flexion du pied porteur lorsque la barrette est retirée du semelage.

En variante non représentée, le semelage de la chaussure thérapeutique peut être formé d'une unique semelle formant une semelle d'usure sur laquelle repose le pied du porteur lorsque la chaussure est portée.

En outre, en variante non représentée, la chaussure thérapeutique selon l'invention peut comporter plusieurs pièces rigides de renfort amovible, chacune de ces pièces étant logée amovible dans le semelage de la chaussure thérapeutique selon une disposition permettant à la pièce de s'opposer à la flexion du semelage dans au moins une direction.

Chacune des pièces rigides de renfort amovible peut être formée d'une barrette rigide, cambrée ou non, d'une tige rigide ou comporter tout autre structure apte à s'opposer à la flexion du semelage de la chaussure dans au moins une direction.

En variante non représentée ou en combinaison, rien n'empêche qu'une pièce rigide de renfort amovible soit logée dans une semelle intérieure plutôt que dans la semelle d'usure. Notamment, une pièce rigide de renfort amovible peut être logée dans une première 1 du semelage, ou dans une semelle intercalaire disposée entre ladite première et la semelle d'usure du semelage, ou dans toute autre semelle intérieure du semelage.

En variante non représentée ou en combinaison, rien n'empêche qu'une pièce 15 rigide de renfort amovible soit logée dans une gorge ménagée dans la surface d'appui du semelage.

En variante non représentée ou en combinaison, rien n'empêche qu'une pièce 15 de renfort amovible soit formée par un organe rigide se fixant latéralement sur un semelage ou une structure rigide pouvant former un cerclage du semelage.

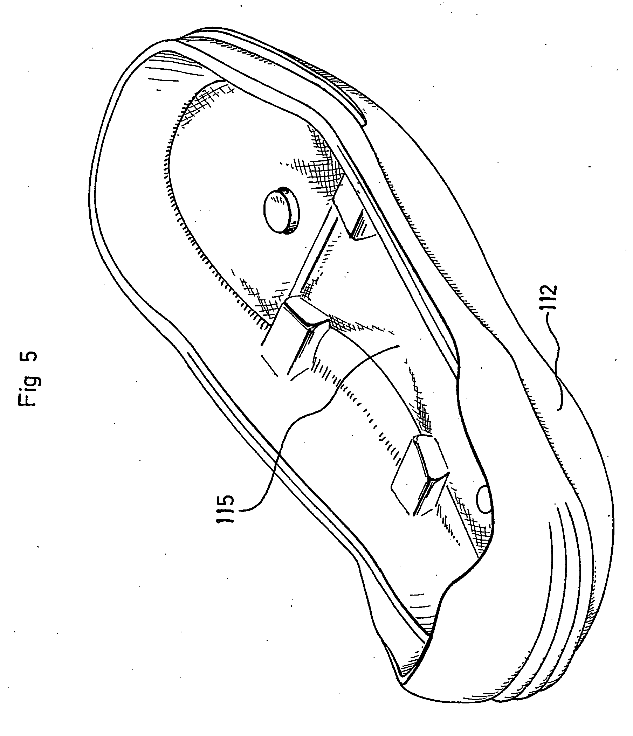

La figure 5 illustre une semelle d'usure d'une chaussure thérapeutique selon un deuxième mode de réalisation de l'invention, similaire à celle du premier mode de réalisation de l'invention sauf en ce que la pièce 115 rigide de renfort amovible est logée entre une face intérieure de la semelle 112 d'usure et une première (non représentée) plutôt que dans un évidemment de la semelle d'usure. La pièce 115 rigide de renfort présente une forme conjuguée de celle de la face intérieure de manière à s'étendre davantage dans la dimension en largeur de la chaussure au niveau d'une partie avant de la chaussure. En outre, la pièce rigide s'étend davantage dans la dimension en longueur de la chaussure, soit en regard d'un emplacement pour talon d'une face supérieure du semelage et jusqu'en regard d'une extrémité antérieure d'un emplacement pour orteils de la face supérieure du semelage. La chaussure thérapeutique selon le mode de réalisation amélioré permet de réaliser une immobilisation encore plus efficace du pied contre des flexions de l'avant-pied dans le sens longitudinal ainsi qu'en vrillage latéral.

L'invention peut faire l'objet d'autres variantes de réalisation par rapport aux modes de réalisation représentés sur les figures et décrits ci-dessus à titre d'exemples non limitatifs.

## Revendications

1. Chaussure thérapeutique comportant un semelage présentant :
- une surface d'appui (6) au sol à profil bombé dans un plan sagittal de la chaussure,
- une surface (17) supérieure comprenant un emplacement pour orteils et un emplacement pour talon,
- le semelage étant conformé pour que le profil bombé de la surface (6) d'appui s'étende en regard de l'emplacement pour talon,
- le semelage étant longitudinalement flexible dans le plan sagittal,
- la chaussure comprenant au moins une pièce (15, 115) rigide de renfort amovible s'opposant à la flexion longitudinale du semelage dans un plan sagittal,
**caractérisée en ce que** :
- le semelage est conformé pour que le profil bombé de la surface (6) d'appui s'étende en regard de l'emplacement pour orteils,
- la pièce (15, 115) rigide de renfort est logée de façon amovible dans un logement du semelage de forme conjuguée de celle de la pièce (15, 115) rigide de renfort, ledit logement étant formé dans une semelle (2, 112) d'usure du semelage et/ou dans une semelle (1) intérieure amovible du semelage reposant au moins en partie sur une face (7) intérieure supérieure de la semelle (2, 112) d'usure, qui est opposée à ladite surface (6) d'appui au sol.

2. Chaussure thérapeutique selon la revendication 1, **caractérisée en ce que** le profil bombé de la surface d'appui (6) s'étend jusqu'en regard d'une extrémité antérieure de l'emplacement pour orteils.

3. Chaussure thérapeutique selon l'une des revendications 1 ou 2, **caractérisée en ce qu'**au moins une pièce (15, 115) rigide est conformée pour s'étendre en regard de l'emplacement pour talon et de l'emplacement pour orteils.

4. Chaussure selon l'une des revendications 1 à 3, **caractérisée en ce que** ledit logement est formé d'un évidement (5) de ladite face (7) intérieure.

5. Chaussure selon l'une des revendications 1 à 4, **caractérisé en ce que** ledit logement est formé dans une première (1) de la chaussure.

6. Chaussure selon l'une des revendications 1 à 5, **caractérisée en ce que** le semelage comporte des moyens de fixation amovible de la semelle intérieure (1) par rapport à la face intérieure (7) de la semelle (2, 112) d'usure, adaptés pour s'opposer à tout glissement longitudinal relatif de la semelle intérieure par rapport à la face intérieure (7) de la semelle (2, 112) d'usure.

7. Chaussure selon la revendication 6, **caractérisée en ce que** la semelle (2, 112) d'usure présente au moins un plot (9) s'étendant vers le haut, chaque plot (9) étant adapté pour pénétrer dans une cavité ménagée dans ladite première (1), le(les) plot(s) (9) et la(les) cavité(s) présentant des formes conjuguées pour former des moyens de fixation amovible.

8. Chaussure selon l'une des revendications 1 à 7, **caractérisée en ce qu'**elle comprend une unique pièce (15, 115) rigide de renfort amovible sous forme d'une plaque (15, 115) s'étendant longitudinalement et horizontalement en regard de la majeure partie de ladite surface d'appui (6).

9. Chaussure selon la revendication 8, **caractérisée en ce que** ladite plaque (15, 115) est cintrée selon un profil arqué avec une courbure dans le même sens que le profil bombé de ladite surface d'appui (6).

10. Chaussure thérapeutique selon l'une des revendications 1 à 9, **caractérisée en ce que** la surface (6) d'appui au sol est exclusivement constituée par une surface d'usure d'une semelle (2, 112) d'usure formée en une pièce unique et s'étend en regard d'une extrémité postérieure de l'emplacement pour talon, jusqu'en regard d'une extrémité antérieure de l'emplacement pour orteils.

## Claims

1. Therapeutic shoe comprising a sole assembly which has:
- a tread surface (6) having a curved profile in a sagittal plane of the shoe,
- an upper surface (17) which comprises a location for the toes and a location for the heel,
- the sole assembly being shaped so that the curved profile of the tread surface (6) extends opposite the location for the heel,
- the sole assembly being longitudinally flexible in the sagittal plane,
- the shoe comprising at least one removable rigid reinforcement component (15, 115) which acts counter to the longitudinal flexion of the sole assembly in a sagittal plane,
**characterised in that**:
- the sole assembly is shaped so that the curved profile of the tread surface (6) extends opposite the location for the toes,
- the rigid reinforcement component (15, 115) is accommodated in a removable manner in a housing of the sole assembly having a shape which corresponds to that of the rigid reinforcement component (15, 115), the said housing being formed in an outer sole (2, 112) of the sole assembly and/or in a removable inner sole (1) of the sole assembly which rests at least partially on an upper inner face (7) of the outer sole (2, 112) which is opposite the said tread surface (6).

2. Therapeutic shoe according to Claim 1, **characterised in that** the curved profile of the tread surface (6) extends as far as a location opposite a front end of the location for the toes.

3. Therapeutic shoe according to one of Claims 1 or 2, **characterised in that** at least one rigid component (15, 115) is shaped so as to extend opposite the location for the heel and the location for the toes.

4. Shoe according to one of Claims 1 to 3, **characterised in that** the said housing is formed by a recess (5) of the said inner face (7).

5. Shoe according to one of Claims 1 to 4, **characterised in that** the said housing is formed in an insole (1) of the shoe.

6. Shoe according to one of Claims 1 to 5, **characterised in that** the sole assembly comprises means for removably fixing the inner sole (1) relative to the inner face (7) of the outer sole (2, 112), which means are capable of acting counter to any relative longitudinal sliding action of the inner sole relative to the inner face (7) of the outer sole (2, 112).

7. Shoe according to Claim 6, **characterised in that** the outer sole (2, 112) has at least one stud (9) which extends upwards, each stud (9) being capable of extending into a cavity which is provided in the said insole (1), the stud(s) (9) and the cavity or cavities having corresponding shapes in order to form removable fixing means.

8. Shoe according to one of Claims 1 to 7, **characterised in that** it comprises a single rigid removable reinforcement component (15, 115) in the form of a plate (15, 115) which extends longitudinally and horizontally opposite the main portion of the said tread surface (6).

9. Shoe according to Claim 8, **characterised in that** the plate (15, 115) is curved in accordance with an arced profile with a curvature in the same direction as the curved profile of the said tread surface (6).

10. Therapeutic shoe according to one of Claims 1 to 9, **characterised in that** the tread surface (6) is exclusively constituted by a tread surface of an outer sole (2, 112) which is formed in one piece and which extends opposite a rear end of the location for the heel, as far as a location opposite a front end of the location for the toes.

## Patentansprüche

1. Therapeutischer Schuh mit einer Besohlung, die folgendes aufweist:
- eine Abstützfläche (6) auf dem Boden, mit in einer pfeilrechten Ebene des Schuhs gewölbtem Profil,
- eine obere Fläche (17) mit einem Sitz für Zehen und einem Sitz für Hacke,
- wobei die Besohlung so ausgebildet ist, dass sich das gewölbte Profil der Abstützfläche (6) gegenüber dem Sitz für Hacke erstreckt,
- wobei die Besohlung in pfeilrechter Ebene in Längsrichtung flexibel ist,
- wobei der Schuh mindestens ein herausnehmbares steifes Verstärkungsteil (15, 115) umfasst, das sich der Längsflexion der Besohlung in einer pfeilrechten Ebene widersetzt,
**dadurch gekennzeichnet, dass**
- die Besohlung so ausgebildet ist, dass sich das gewölbte Profil der Abstützfläche (6) gegenüber dem Sitz für Zehen erstreckt,
- das steife Verstärkungsteil (15, 115) herausnehmbar in einer Aufnahme der Besohlung gelagert ist, deren Form diejenige des steifen Verstärkungsteils (15, 115) ergänzt, wobei die genannte Aufnahme in einer Verschleißsohle (2, 112) der Besohlung und/oder in einer herausnehmbaren Innensohle (1) der Besohlung ausgebildet ist, die zumindest teilweise auf einer oberen Innenseite (7) der Verschleißsohle (2, 112) ruht, die der genannten Abstützfläche (6) auf dem Boden gegenüberliegt.

2. Therapeutischer Schuh gemäß Anspruch 1, **dadurch gekennzeichnet, dass** sich das gewölbte Profil der Abstützfläche (6) bis gegenüber einem dem Sitz für Zehen vorgelagerten Ende erstreckt.

3. Therapeutischer Schuh gemäß einem der Ansprüche 1 oder 2, **dadurch gekennzeichnet, dass** mindestens ein steifes Teil (15, 115) ausgebildet ist, um sich gegenüber dem Sitz für Hacke und dem Sitz für Zehen zu erstrecken.

4. Schuh gemäß einem der Ansprüche 1 bis 3, **dadurch gekennzeichnet, dass** die genannte Aufnahme in einer Aussparung (5) der genannten Innenseite (7) ausgebildet ist.

5. Schuh gemäß einem der Ansprüche 1 bis 4, **dadurch gekennzeichnet, dass** die genannte Aufnahme in einer ersten Sohle (1) des Schuhs ausgebildet ist.

6. Schuh gemäß einem der Ansprüche 1 bis 5, **dadurch gekennzeichnet, dass** die Besohlung herausnehmbare Befestigungsmittel der Innensohle (1) in Bezug auf die Innenseite (7) der Verschleißsohle (2, 112) umfasst, die geeignet sind, sich jeglichem relativem Längsgleiten der Innensohle in Bezug auf die Innenseite (7) der Verschleißsohle (2, 112) zu widersetzen.

7. Schuh gemäß Anspruch 6, **dadurch gekennzeichnet, dass** die Verschleißsohle (2, 112) mindestens einen sich nach oben erstreckenden Nocken (9) aufweist, wobei jeder Nocken (9) in einen in der genannten ersten Sohle (1) vorgesehenen Hohlraum eindringen kann, wobei die Nocke(n) (9) und der (die) Hohlräum(e) sich ergänzende Formen aufweisen, um abnehmbare Befestigungsmittel zu bilden.

8. Schuh gemäß einem der Ansprüche 1 bis 7, **dadurch gekennzeichnet, dass** er ein einziges herausnehmbares steifes Verstärkungsteil (15, 115) umfasst, in Form einer Platte (15, 115), die sich in Längs- und Horizontalrichtung gegenüber dem größten Teil der genannten Abstützfläche (6) erstreckt.

9. Schuh gemäß Anspruch 8, **dadurch gekennzeichnet, dass** die genannte Platte (15, 115) gemäß einem bogenförmigen Profil gekrümmt ist, mit einer Krümmung in der gleichen Richtung wie das gewölbte Profil der genannten Abstützfläche (6).

10. Therapeutischer Schuh gemäß einem der Ansprüche 1 bis 9, **dadurch gekennzeichnet, dass** die Abstützfläche (6) auf dem Boden ausschließlich aus einer Verschleißfläche einer Verschleißsohle (2, 112) gebildet wird, die aus einem einzigen Teil besteht und sich gegenüber einem dem Sitz für Hacke nachgelagerten Ende erstreckt, bis gegenüber einem dem Sitz für Zehen vorgelagerten Ende.
